Europäisches Patentamt

European Patent Office

Office européen des brevets

(11) Publication number: 0 370 256 A1

# EUROPEAN PATENT APPLICATION

(21) Application number: 89119781.6

(22) Date of filing: 24.10.89

(51) Int. Cl.5: C12P 21/00, C12N 15/00, C12N 5/00, C07K 15/00, //G01N33/92,A61K39/395, (C12P21/00,C12R1:91)

Claim for the following Contracting State: ES.

The microorganism has been deposited with American Type Culture Collection (ATCC) under number: HB-9655.

The applicant has filed a statement in accordance with Rule 28 (4) EPC (issue of a sample only to an expert). Accession number(s) of the deposit(s): ATCC HB-9655.

(30) Priority: 24.10.88 JP 267541/88

(43) Date of publication of application: 30.05.90 Bulletin 90/22

(84) Designated Contracting States: AT BE CH DE ES FR GB GR IT LI LU NL SE

(71) Applicant: MECT CORPORATION 2-1-1, Nishi-Shinjyuku Shinjyuku-ku Tokyo(JP)

(72) Inventor: Nagai, Yoshitaka 1-29-10, Akatsutsumi Setagaya-ku Tokyo(JP)
Inventor: Yamamoto, Hideki 870-17, Kamitsuchidana Ayase-shi Kanagawa-ken(JP)
Inventor: Ito, Masayoshi 1-27-22-303, Fujimidai Kunitachi-shi Tokyo(JP)
Inventor: Tomita, Kenkichi 504, Meguro-Coporasu 3-24-14, Shimomeguro Meguro-ku Tokyo(JP)

(74) Representative: Vossius & Partner Siebertstrasse 4 P.O. Box 86 07 67 D-8000 München 86(DE)

(54) Monoclonal antibody specifically recognizing sulfated glycolipids.

(57) A monoclonal antibody exhibits specificity to sulfated glycolipids, in particular to cerebroside sulfuric acid ester. The monoclonal antibody can be obtained from a hybridoma obtained by fusing (A) an antibody-producing cell obtained by immunizing an animal with a sulfated glycolipid as an antigen, with (B) a myeloma cell. The monoclonal antibody can be used to diagnose renal diseases of animals and treat patients suffering from such diseases. Also, it can be used in the affinity chromatography for purifying antigens capable of bonding thereto.

**Monoclonal Antibody Specifically Recognizing Sulfated Glycolipids**

BACKGROUND OF THE INVENTION

(Field of the Invention)

The present invention relates to a monoclonal antibody and more specifically to a monoclonal antibody which does not react with neutral glycolipids such as Gal-Cer or Glc-Cer, but specifically reacts with sulfated glycolipids.

(Description of the Prior Art)

In the nerves and the brain of animals, sulfuric acid esters of cerebrosides are present as sulfated glycolipids as well as cerebrosides and the white substance of the brain contains a relatively large amount of the cerebroside sulfuric acid esters.

The cerebroside sulfuric acid ester has a sulfate residue bound to the galactose of the cerebroside at the C-3 position through an ester bond. This compound was identified by J.W. Thudichum as a sulfur-containing lipid present in the brain. In 1933, it was defined by G. Blix to be a cerebroside esterified with sulfuric acid. Its final chemical structure was determined by Tamio YAMAKAWA in 1962.

The cerebroside sulfuric acid ester is an important constitutive component of myelin and is specifically bound to a basic protein in myelin. In addition, the cerebroside sulfuric acid ester is also locally present in kidney and it is assumed that the ester is involved in electrolyte ion transport. It is therefore desirable to develop monoclonal antibodies which have high antigenic specificity to cerebroside sulfuric acid esters, which can hence be used in the serodiagnosis and tissue diagnosis for the detection of abnormalities in the electrolyte transport due to renal diseases and which, as a result, can be used for treating such diseases.

SUMMARY OF THE INVENTION

Accordingly, an object of the present invention is to provide a monoclonal antibody exhibiting high antigenic specificity to sulfated glycolipids such as cerebroside sulfuric acid esters (hereinafter referred to as "CSE").

Another object of the present invention is to provide a hybridoma capable of producing a monoclonal antibody specific to the sulfated glycolipids.

A further object of the present invention is to provide a method for establishing a hybridoma capable of producing a monoclonal antibody specific to the sulfated glycolipids.

According to the present invention, 1) a monoclonal antibody exhibiting high antigenic specificity to sulfated glycolipids; 2) a hybridoma capable of producing a monoclonal antibody specific to sulfated glycolipids; and 3) a method for establishing a hybridoma capable of producing a monoclonal antibody specific to sulfated glycolipids which comprises the step of cell-fusing (A) antibody-producing cells obtained by immunizing an animal with sulfated glycolipids as an antigen and (B) myeloma cells, are provided.

BRIEF EXPLANATION OF THE DRAWINGS

Fig. 1 is a graph showing the elution behavior of the hybridoma culture medium by an affinity chromatography technique;

Fig. 2 is a diagram showing a pattern of the sedimentation line as measured by the immunodiffusion technique;

Fig. 3a and 3b are diagrams showing patterns of the TLC analysis and orcinol staining analysis; and

Figs. 4 and 5 are diagrams showing the reactivity of the monoclonal antibody of the present invention.

DETAILED EXPLANATION OF THE INVENTION

The present invention will be explained in more detail below.

The monoclonal antibody of the present invention does not react with neutral glycolipids, but specifically reacts with sulfated glycolipids.

The monoclonal antibody of the present invention can be, for instance, obtained according to the method of Köhler & Milstein. More specifically, the method comprises fusing an antibody-producing cell derived from an animal immunized with the sulfated glycolipids as an antigen, with myeloma cells, using an agent for cell fusion such as polyethylene glycol (PEG); selecting only hybridomas capable of producing monoclonal antibodies having specificity to the antigen; proliferating the hybridomas and purifying the monoclonal antibodies produced by the hybridomas.

The immunization of an animal like a mouse is performed by intraperitoneal, subcutaneous or intravenous injection of the CSE. Preferable is intravenous injection of the CSE. In the immunization, complete Freund's adjuvant or incomplete Freund's adjuvant may be used as an adjuvant, i.e., an agent for immunological enhancement. The adjuvant includes oils, emulsifying agents, killed tubercule bacillus, killed Salmonella and mixtures thereof. Preferable adjuvants includes killed Salmonella, in particular killedSalmonella minesota for intraperitoneal or subcutaneous injection. These adjuvants and the CSE as an antigen are preferably used in the form of a solution having an approximately physiologically acceptable composition such as a solution in phosphate-buffered saline.

The animal to be immunized with the foregoing CSE as the antigen may be any kind of animal and examples include rabbit, man, mouse and rat, preferably a mouse and more preferably a Balb/c mouse is used. The antibody-producing cells are in general derived from the spleen of the animal.

The myeloma cells to be used in the present invention may be derived from an animal such as those listed above, preferably those derived from a mouse, and more preferably myeloma cells X63-Ag8-6.5.3 derived from a Balb/c mouse. These myeloma cells are extremely proliferative and, therefore, can impart vigorous proliferation activity to the resulting hybridoma.

Various agents such as HJV can be used as well as polyethylene glycol as an agent for cell fusion. The degree of polymerization of polyethylene glycol ranges from 2,000 to 10,000, preferably approximately 4,000.

Various methods for selectively culturing the hybridoma are known and any method may be used in the method of this invention. In this case, HAT medium is generally used as a culture medium. HAT medium into which insulin is incorporated can also be used. Moreover, a culture medium free of serum may be used.

A cloning method for selecting only the hybridomas capable of producing a desired monoclonal antibody is also known and, for instance, the methyl cellulose method, the soft agarose method or the limiting dilution method can be used in the present invention.

The monoclonal antibody according to the present invention can be used in the diagnosis of renal diseases of animals by, for instance, ELISA and RIA assays. Moreover, the monoclonal antibody of the present invention may be used in affinity chromatography for purifying antigens capable of binding thereto. If the monoclonal antibody is labeled with a radioactive isotope, it can be used to detect the renal disease or to locate such portions. It can be used in a high dose to treat patients suffering from such a disease. It is also expected that the monoclonal antibody of the present invention can be used for basic studies of nerve cells and for various clinical purposes.

The present invention will be explained in more detail with reference to the following working Examples, but the scope of the present invention is not restricted to the specific Examples.

Example

(1) Extraction and Purification of CSE

Excess fats and oils were removed from hog adrenal gland. The hog adrenal gland was then homogenized in cold acetone and dried to obtain a powder. The hog adrenal gland powder was extracted with a mixed solvent of chloroform, methanol and water and the extract was purified utilizing an Iatrobeads column and a DEAE-Sephadex column.

In this case, gangliosides $GM_c$, $GD_3$ and $Gm_2$ available from FUNAKOSHI Company were used as such without further purification. Sulfated cholesterol available from SIGMA Company was used as such without further purification.

These ganglioside glycolipids were dissolved in a chloroform-methanol [1:1 (v/v) ] mixture or ethanol

3

and stored at a temperature of -20 ° C.

### (2) Preparation of Antigen Solution

The purified CSE extracted from hog adrenal gland and Salmonella minesota R 595 treated with acetic acid were mixed in phosphate-buffered saline [PBS(-) CSE 5 μ g/100 μ ℓ] at a weight ratio of 1:10. The resulting solution was used as an antigen solution.

### (3) Animals to be Immunized

Female Balb/c mice (6-week-old) were used for the following experiments after they were bred under ordinary conditions.

### (4) Culture Medium

Culture Medium: Nissui RPMI 1640 was used as a culture medium. To the culture medium, kanamycin sulfate and fetal bovine serum (FBS) were added so that the final concentrations thereof were equal to 50 μ g/m ℓ and 10% respectively prior to use.

HAT Medium: 0.0388 g of thymidine and 0.1361 g of hypoxanthine were dissolved in 100 mℓ of distilled water while heating. The resulting solution (a) having a concentration 100 times higher than the desired one was stored at -20 °C as a stock solution. Likewise, 0.0176 g of aminopterin was dissolved in 100 mℓ of distilled water by adding a small amount of a 1N sodium hydroxide aqueous solution to it. Then, the solution was diluted 10 times with a RPMI 1640 culture medium. The resulting solution (b) was stored at -20 °C, shielding from light, as a stock solution having a concentration 100 times higher than the desired one. The HAT medium was prepared by adding 1/100 volume each of these two solutions (a) and (b) to a 10% FBS RPMI 1640 medium immediately before use.

Moreover, HT medium was prepared by simply adding 1/100 volume of the stock solution (a) containing hypoxanthine and thymidine to the same 10% FBS RPMI 1640 medium.

### (5) Parent Cells

As parent cells for cell fusion, myeloma cells (X63-Ag8-6.5.3 cells) were used which had been derived from Balb/c mice. These cells were cultured in an RPMI 1640 medium containing 10% FBS and 6-thioguanine at 3 μ g/m ℓ.

### (B) Preparation of Hybridoma

### (1) Method of Immunization

The aforesaid 6-week-old female Balb/c mice were immunized by intravenously injecting the foregoing antigen solution according to the following immunization schedule: 5 μ g each at 0, 10 and 17 days from the initial immunization. 20 Days after the initial immunization the spleens were dissected from the mice and a suspension containing separately dispersed spleen cells was prepared and subsequently used in the cell fusion.

### (2) Cell Fusion

The spleen cells (lymphocytes) prepared above were fused with the mouse myeloma cells, i.e., the foregoing parent cells (X63-Ag8 -6.5.3. cells) according to the method of Kohler & Milstein. More specifically, 1 X 10^8 of the spleen lymphocytes were fused with 2 x 10^7 of the mouse myeloma cells (X63-Ag8-6.5.3. cells) in the presence of 50% polyethylene glycol (PEG 4,000) in a culture medium.

### (3) Selection of Hybridoma

After the cell fusion, the resultant cells were cultured at 37°C in HAT medium in the presence of 5% $CO_2$ to select and culture hybridoma.

### (4) Selection of Antibody-Producing Cells

The selected hybridoma was dispensed in a 96-well microplate to culture the same. Among these, the cells capable of producing an intended antibody were selected by the enzyme-linked immunosorbent assay method (ELISA method). This procedure was repeated to obtain an antibody-producing cell line named 4E11. The hybridoma (cell line) thus obtained was deposited with Americal Type Culture Collection (ATCC) under the accession number of HB 9655.

The ELISA method adopted herein was carried out as follows:

### Enzyme-linked Immunosorbent Assay (ELISA Method)

A 96-well flat bottomed plate (available from Falcon Co., Ltd.) was pretreated with ethanol before using it in experiments. 50 $\mu l$ of the antigen solutions which had been diluted with ethanol to adjust the concentration of the CSE to 20 $\mu$ g/m $l$ (optimum conc.) was pipetted into each well of the plate; the solvent was evaporated off; then 100 $\mu l$ of a 1% ovalbumin PBS(-) solution was introduced into each well, which were allowed to stand for 30 minutes at room temperature. The plate was dumped down and shaken to remove the excess solution. Then, 50 $\mu l$ of the supernatant of the hybridoma culture media, as a primary antibody, was added to each well, which were allowed to stand for one hour and 30 minutes at room temperature. Likewise, the primary antibody was removed from the wells. Then, the wells were washed 3 times by adding 150 $\mu l$ of a PBS(-) solution. 100 $\mu l$ of a 1% ovalbumin- PBS(-) solution was added to the wells which were allowed to stand at room temperature for 30 minutes. After the removal of the excess solution, 50 $\mu l$ of a secondary antibody, which had been diluted with a 1% ovalbumin- PBS(-) solution to its optimum concentration [goat anti-mouse IgG, M and A antibodies labeled with horseradish peroxidase (HRP) ] , was added to the wells which were left to stand for one hour and a half at room temperature. As in the case of the primary antibody, the wells were washed 3 times with a PBS(-) solution. 100$\mu l$ of a reaction solution was added to the wells to cause the reaction in the dark. The reaction solution was prepared by dissolving o-phenylenediamine dichloride and hydrogen peroxide in a citrate-phosphate buffer (pH = 5) so that the concentrations thereof were 0.4 mg/m $l$ and 0.01%, respectively. The reaction was stopped by adding 30 $\mu l$ of an 8N sulfuric acid solution to the wells. The product was examined by colorimetry at 500 nm.

### (C) Monoclonal Antibody

### (1) Purification of Monoclonal Antibody

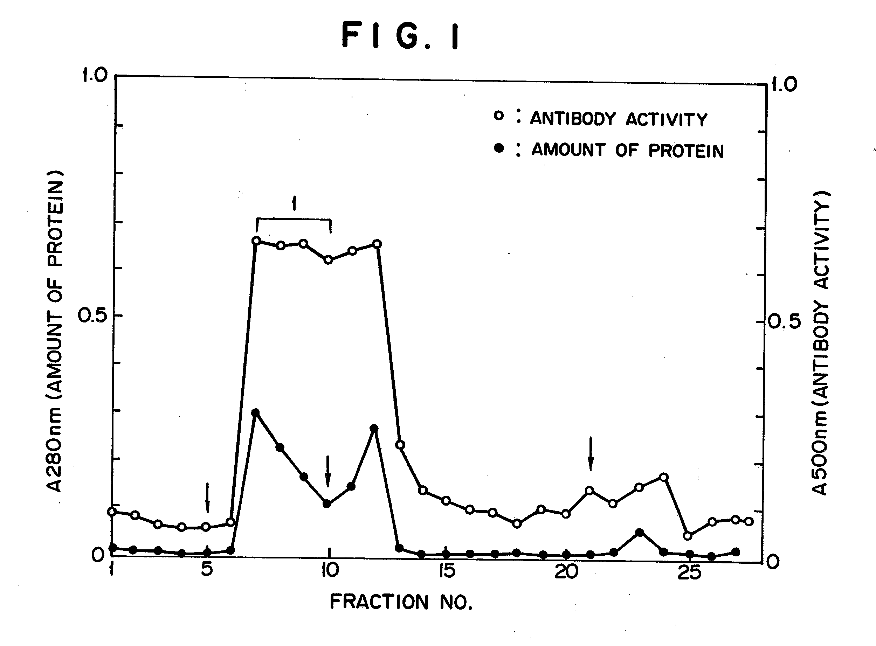

A culture of the hybridoma was passed through an affinity chromatography column packed with Protein A Sepharose to adsorb antibodies to the column and then the column was stepwise eluted with PBS solutions having a pH of 7.0, 5.5, 4.3 and 2.3, respectively. The results observed are plotted in Fig. 1. In Fig. 1, elute 1 having high antibody activity per unit amount of protein was obtained as a high activity function.

### (2) Identification of Antigen and Investigation of Specificity of the Antibody

### A. Qualitative Immunodiffusion Technique (Ouchterlony Method)

The class of the monoclonal antibody in the high activity fraction was determined according to the known qualitative immunodiffusion technique.

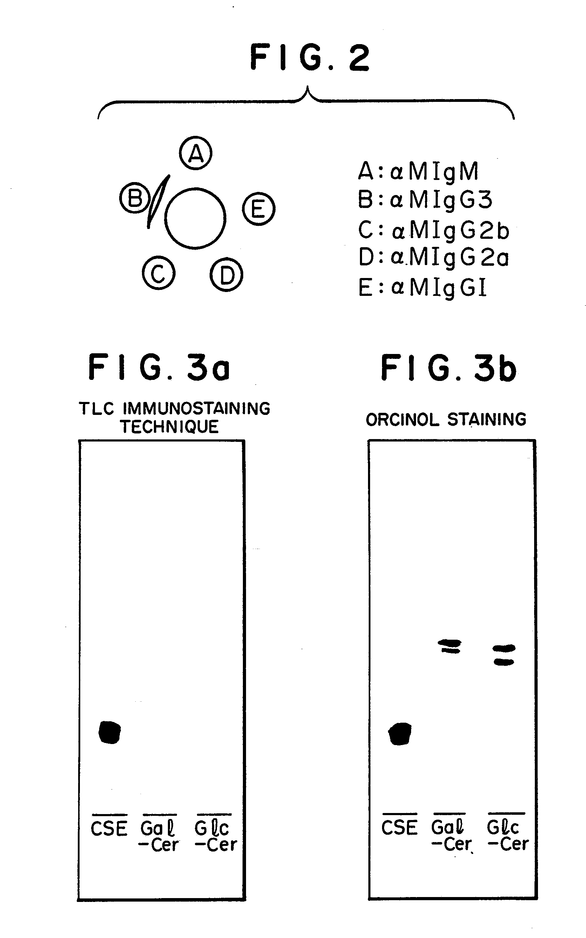

Fig. 2 shows the results by the known qualitative immunodiffusion technique. In Fig. 2, αMIgM, αMIgGi, G$_{2a}$, Gβ$_b$ , and Gç are anti-sera obtained from MINUS Company, respectively. The results in Fig. 2 show that the monoclonal antibody of the present invention is IgGç.

B. ELISA Method

According to the foregoing procedures, the reactivity of the monoclonal antibody of the present invention with CSE and each glycolipid were investigated while maintaining the amounts of the antigen and antibody constant (using the hybridoma culture medium for the CSE as an antigen). The results are listed in the following Table I.

Table I

| Antigen (1 μ g/well) | Absorption at OD$_{50\ 0nm}$ |
|---|---|
| CSE | 1.237 ± 0.017 |
| GMç | 0.098 ± 0.004 |
| GDç | 0.101 ± 0.010 |
| GMβ | 0.107 ± 0.015 |
| Free of Antigen | 0.149 ± 0.010 |

From the results of Table I, it is found that the monoclonal antibody of the present invention exhibits strong specificity for CSE.

C. TLC Immunostaining Technique

A silica gel thin layer chromatographic [TLC(poly gram SIL G)] plate was cut into two pieces of an appropriate size and to each a small drop of the solution of a glycolipid in chloroform-methanol [1:1 (v/v)] was applied. Depending on the purpose, the samples were developed with a developing solution such as chloroform-methanol-water [60/40/10 (v/v)] , chloroform-methanol-0.5% CaCl$_2$ solution [55/45/10 (v/v)] or chloroform-methanol-2.5N NH$_4$OH solution [60/40/9 (v/v) ] and were then allowed to stand in a 1% ovalbumin-1% polyvinyl pyrrolidone (k-30) PBS(-) solution at 40° C over night. Then, one of the samples was dipped in the primary antibody (not purified) solution and then shaken for 2 hours at room temperature. After sufficiently washing it with PBS(-), it was dipped into 1% ovalbumin-1% polyvinyl pyrrolidone (k-30) PBS(-) solution at room temperature for 30 minutes. The sample was withdrawn therefrom,and sufficiently dipped in the secondary antibody which had been diluted to its optimum concentration with a 3% polyvinyl pyrrolidone (k-30) PBS(-) solution, shaken for 2 hours at room temperature, then sufficiently washed with PBS(-). A reaction solution was added to the sample. The reaction solution was prepared by dissolving 4 mg of 4-chloro-1-naphthol in 1 ml of methanol, adding 50 mmole of tris(hydroxymethyl) -aminomethane, 200 mmmole of NaCl and 5 ml of a buffer (pH = 7.4) and then adding hydrogen peroxide so that the concentration thereof was 0.01%. The reaction was stopped by washing the plate with water followed by air-drying. (Fig. 3a)

On the other hand, the other sample was stained with orcinol (Fig. 3b).

From Fig. 3a and 3b,it may be seen that the monoclonal antibody of the present invention quite specifically reacts with CSE.

In addition, to confirm the reactivity of the monoclonal antibody of the present invention, the following investigation was performed utilizing the purified monoclonal antibody.

D. Reactivity 1

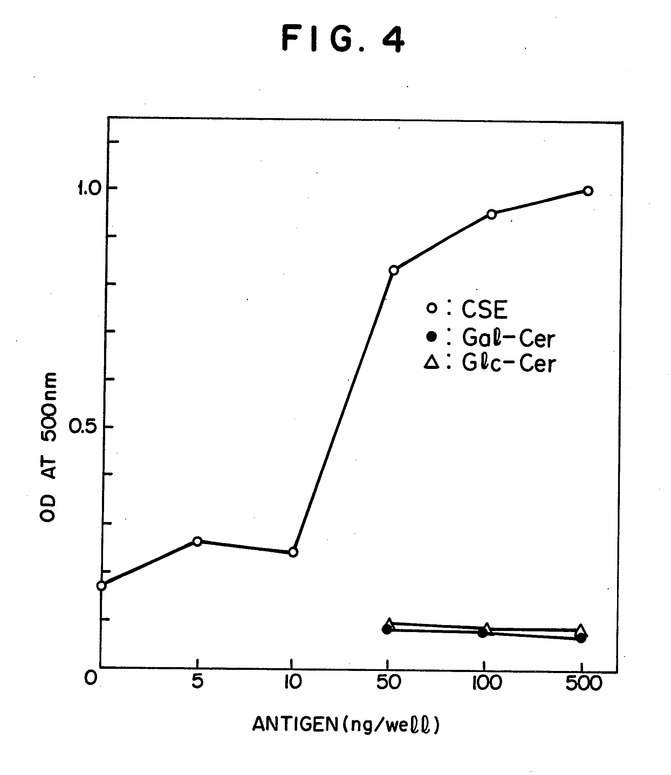

The reactivity of the monoclonal antibody of the present invention was investigated. The amount of the antigen was changed while the amount of the purified monoclonal antibody was maintained constant (50 ng/well). Moreover, Gal-Cer and Glc-Cer (neutral glycolipids) were also used as an antigen as well as the CSE. The reactivity of the antibody with these neutral glycolipids was also examined. The results thus observed are plotted in Fig. 4.

From Fig. 4, it may be seen that the monoclonal antibody of the present invention does not react with the neutral glycolipids Gal-Cer and Glc-Cer. Further, the antigen in an amount up to 50 ng/well can be detected easily.


E. Reactivity 2

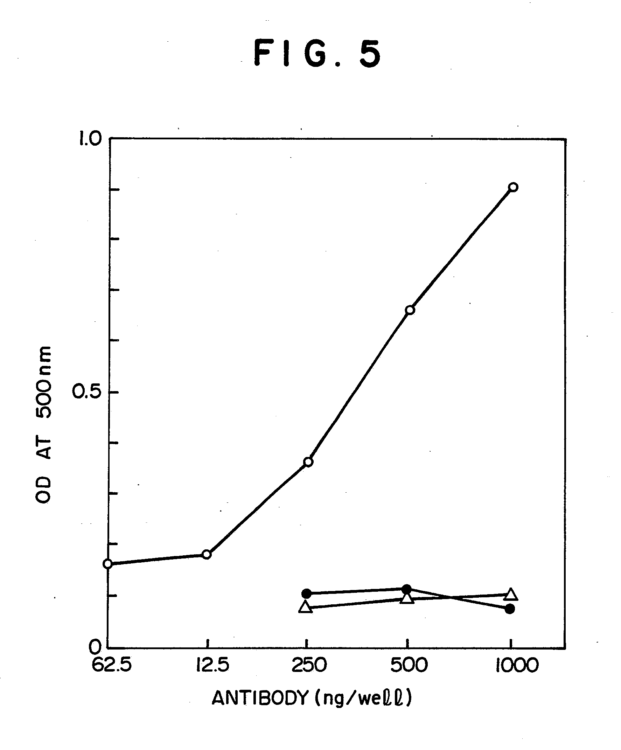

The reactivity of the monoclonal antibody of the present invention was investigated by changing the amount of the monoclonal antibody and maintaining the amount of the antigen constant (500 ng/well). The results thus obtained are shown in the attached Fig. 5.

From Fig. 5, it may be seen, as in the investigation of the reactivity 1, that the monoclonal antibody of the present invention exhibits high specificity to the CSE. It is also found that the limit of detection clearly increases as the amount of the monoclonal antibody increases.


**Claims**

1. A monoclonal antibody exhibiting specificity to sulfated glycolipids.

2. The monoclonal antibody of claim 1 wherein the sulfated glycolipid is a cerebroside sulfuric acid ester.

3. The monoclonal antibody of claim 1 wherein it is IgGç.

4. The monoclonal antibody of claim 1 produced by a hybridoma obtained by cell fusion between spleen cells of Balb/c mouse and myeloma cells, X63-Ag8-6.5.3.

5. The monoclonal antibody of claim 4 wherein the hybridoma is a cell line deposited with ATCC under the accession number of HB 9655.

6. A hybridoma capable of producing a monoclonal antibody exhibiting specificity to sulfated glycolipid.

7. The hybridoma of claim 6 obtained by cell fusion between spleen cells of Balb/c mouse and myeloma cells, X63-Ag8-6.5.3.

8. The hybridoma of claim 7 deposited with ATCC under the accession number of HB 9655.

9. A method for producing a hybridoma capable of producing a monoclonal antibody exhibiting specificity to sulfated glycolipids comprising fusing (A) an antibody-producing cell obtained by immunizing an animal with a sulfated glycolipid as an antigen, with (B) a myeloma cell.

10. The method of claim 9 wherein the sulfated glycolipid is a cerebroside sulfuric acid ester.

11. The method of claim 10 wherein the cerebroside sulfuric acid ester is derived from hog adrenal glands.

12. The method of claim 9 wherein an adjuvant selected from the group consisting of oils, emulsifying agents, killed tubercule bacillus, killed Salmonella and a mixture thereof is used with the sulfated glycolipid.

13. The method of claim 12 wherein the adjuvant is killed Salmonella minesota.

14. The method of claim 9 wherein the immunization is performed by intravenous injection.

15. The method of claim 9 wherein the animal is Balb/c mouse and the antibody-producing cell is a spleen cell of the Balb/c mouse.

16. The method of claim 15 wherein the myeloma cell is derived from Balb/c mouse.

17. The method of claim 16 wherein the myeloma cell is X63-Ag8-6.5.3. cell.

18. The method of claim 9 wherein polyethylene glycol having a degree of polymerization ranging from 2,000 to 10,000 is used as an agent for cell fusion.

Claims for the following Contracting State: ES

1. A method for producing a hybridoma capable of producing a monoclonal antibody exhibiting specificity to sulfated glycolipids comprising fusing (A) an antibody-producing cell obtained by immunizing an animal with a sulfated glycolipid as an antigen, with (B) a myeloma cell.

2. The method of claim 1 wherein the sulfated glycolipid is a cerebroside sulfuric acid ester.

3. The method of claim 2 wherein the cerebroside sulfuric acid ester is derived from hog adrenal glands.

4. The method of claim 1 wherein an adjuvant selected from the group consisting of oils, emulsifying agents, killed tubercule bacillus, killed Salmonella and a mixture thereof is used with the sulfated glycolipid.

5. The method of claim 4 wherein the adjuvant is killed Salmonella minesota.

6. The method of claim 1 wherein the immunization is performed by intravenous injection.

7. The method of claim 1 wherein the animal is Balb/c mouse and the antibody-producing cell is a spleen cell of the Balb/c mouse.

8. The method of claim 7 wherein the myeloma cell is derived from Balb/c mouse.

9. The method of claim 8 wherein the myeloma cell is X63-Ag8-6.5.3. cell.

10. The method of claim 1 wherein polyethylene glycol having a degree of polymerization ranging from 2,000 to 10,000 is used as an agent for cell fusion.

11. A method of producing a monoclonal antibody exhibiting specificity to sulfated glycolipids which comprises culturing the hybidoma obtained according to any one of claims 1 to 10 and selecting the cells producing the intended antibody.

12. A method according to claim 11 wherein the sulfated glycolipid is cerebroside sulfuric acid ester.

13. A method according to claims 11 and 12 wherein the monoclonal antibody is IgG$_c$.

14. A method according to claims 11-13 wherein the antibody is obtained from a hybridoma deposited with ATCC under the accession number of HB 9655.

# F I G. I

EP 0 370 256 A1

# F I G. 2

A : αMIgM
B : αMIgG3
C : αMIgG2b
D : αMIgG2a
E : αMIgGI

# F I G. 3a

TLC IMMUNOSTAINING
TECHNIQUE

$\overline{CSE}$    $\overline{Gal}$    $\overline{Glc}$
        $-Cer$   $-Cer$

# F I G. 3b

ORCINOL STAINING

$\overline{CSE}$    $\overline{Gal}$    $\overline{Glc}$
        $-Cer$   $-Cer$

# FIG. 4

# F I G. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 108, no. 19, 9th May 1988, page 505, abstract no. 165846s, Columbus, Ohio, US; P. FREDMAN et al.: "Characterization of the binding epitope of a monoclonal antibody to sulfatide", & BIOCHEM. J. 1988, 251(1), 17-22 * Whole abstract * | 1-2,4,6 -13 | C 12 P 21/08 // G 01 N 33/92 (C 12 P 21/00 C 12 R 1:91 ) |
| X | CHEMICAL ABSTRACTS, vol. 101, no. 11, 10th September 1984, page 464, abstract no. 88586h, Columbus, Ohio, US; W. HOFSTETTER et al.: "Production and characterization of monoclonal antibodies to the myelin glycolipid sulfatide", & J. NEUROSCI. RES. 1984, 11(4), 341-50 * Whole abstract * | 1-2,11-12 | |
| X | CHEMICAL ABSTRACTS, vol. 109, no. 17, 24th October 1988, page 373, abstract no. 145685f, Columbus, Ohio, US; A. SCICUTELLA et al.: "Characterization of monoclonal antibodies to galactolipids and uses in studies of dementia", & J. NEUROPATHOL. EXP. NEUROL. 1988, 47(4), 406-19 * Whole abstract * | 1-2 | TECHNICAL FIELDS SEARCHED (Int. Cl.5) C 12 P |

The present search report has been drawn up for all claims

| Place of search | Date of completion of the search | Examiner |
|---|---|---|
| THE HAGUE | 26-02-1990 | ALVAREZ Y ALVAREZ C. |

EPO FORM 1503 03.82 (P0401)